# EUROPEAN PATENT SPECIFICATION

(11) **EP 2 328 561 B1**
(45) Date of publication and mention of the grant of the patent: **25.11.2015**
(21) Application number: 09786149.6
(22) Date of filing: 18.08.2009
(51) Int. Cl.: A61K 9/20, A61K 31/135, A61K 31/167, A61K 31/196

(54) **Rate Modulated Delivery of Drugs from a Three-Layer Tablet Comprising Tramadol, Diclofenac, Paracetamol**
Freisetzung von Wirkstoffen in modulierten Schüben aus einer Dreischichttablette enthaltend Tramadol, Diclofenac, Paracetamol
Livraison modulée en phases d'agents actifs d'un comprimé à trois couches contenant tramadole, diclofénac, paracètamole

(30) Priority: 19.08.2008 ZA 200807122
(43) Date of publication of application: 08.06.2011
(73) Proprietor: Adcock Ingram Intellectual Property (Pty) Limited, Midrand 1685 (ZA)
(72) Inventor: HOBBS, Kim, Mellssa, Johannesburg (ZA); PILLAY, Viness, Sandton 2196 (ZA); CHOONARA, Yahya, Essop, Lenasia 1820 (ZA); PARSONS, Bradley, Ryan, Cape Town (ZA)
(74) Representative: Boult Wade Tennant
(86) International application number: PCT/IB2009/006574
(87) International publication number: WO 2010/020856

(56) References cited:
- WO-A1-2004/054571
- WO-A1-2005/030181
- WO-A1-2008/097066
- WO-A2-2008/015221

## Description

### FIELD OF THE INVENTION

This invention relates to a multi-configured pharmaceutical dosage form and, more particularly, to a multi-layered tablet pharmaceutical dosage form or various multi-unit formulations suitable for the rate-modulated delivery of single or multiple pharmaceutical compositions.

### BACKGROUND TO THE INVENTION

With pain management, it is necessary to develop methods of facilitating treatments that promote compliance with prescriptions and simplify prescribing without increasing adverse effects. Poly-pharmacy is seen as a barrier to prescription compliance and highlights a need for the development of fixed dose combinations which allow the number of tablets taken daily to be reduced, but with no loss in efficacy or an increase in the incidence of side effects. The expected benefits of analgesic combinations include reduced onset of action, increased duration of action, improved efficacy, reduced opioid intake and reduced adverse reactions.

The combining of analgesic drugs with differing mechanisms of nociceptive pain modulation offers benefits including synergistic analgesic effects where the individual agents or components of a therapeutic composition act in a greater than additive manner, and a reduced incidence of side effects. The combinations are most effective when the individual agents act *via* unique analgesic mechanisms and act synergistically by inhibiting multiple pain pathways. This multimodal coverage offers more effective relief for a broader spectrum of pain. Opioids are considered first line medication for relieving severe nociceptive pain but are inadequate in controlling dynamic pain as well being associated with significant side effects. Alternative pain relief using non-opioid analgesics historically relied on paracetamol supplemented with non-steroidal anti-inflammatory drugs (NSAIDs).

Analgesic superiority of a fixed dose combination of paracetamol and tramadol over either individual component, without an increase in side effects has been shown. The fixed combination allows for a reduction in the dose of tramadol, and thereby a reduction in its associated adverse effects, with an equivalent level of analgesia. Data demonstrates that rather than being additive in therapeutic effect, such combinations are, in fact, synergistic.

In a recent study, a codeine/paracetamol/ibuprofen combination was compared against a tramadol/paracetamol combination for the total pain relief that occurred and the sum of the pain intensity differences. During the five- and six-hour assessments of this study the triple combination, that included a different opioid and NSAID than those proposed, showed significant superiority. The vast improvement in duration of action observed four to six hours post-dosing was thought to be due to the anti-inflammatory component.

A pharmacokinetic explanation for this may have been observed in a study which showed that diclofenac transiently reduced the glomerular excretion of the active codeine metabolites, by decreasing prostacyclin production and reducing renal blood flow. This addition of diclofenac to paracetamol and codeine, significantly prolonged the time until analgesic rescue medication was required. No renal pathology is anticipated for the combined used of tramadol and diclofenac as the parenteral combination was tolerated similarly as well as diclofenac or tramadol alone and with no significant increases in side effects compared with placebo dosing, when used for pain in a recent study.

United States Patent 5,516,803 describes a composition of tramadol and a NSAID. In a study using tramadol and ibuprofen on the acetylcholine-induced abdominal constriction in mice, the combination resulted in unexpected analgesic activity enhancement. It was postulated from these results that other NSAIDs, when combined with tramadol, would show similar synergistic activity.

As referenced in United States Patent 6,558,701, describing a multilayer tablet for the administration of a fixed combination of tramadol and diclofenac, the World Health Organisation recommends combining opioid analgesics with NSAIDs for the treatment of moderate to severe pain. The invention of a parenteral suspension of a salt of tramadol and diclofenac, shown in beagle dogs to retard the metabolism of tramadol and thereby prolong analgesia, is described in United States Patent 6,875,447.

The fixed combination of tramadol and paracetamol in Tramacet^{™} (Janssen-Cilag Ltd.) has proved to be a therapeutic advantage and the efficacy of both these active pharmaceutical ingredients seems to benefit from the addition of a NSAID according to the above-cited research. United States Patent 5,516,803 describes the super-additive advantage gained by combining tramadol and a NSAID and two other patents describe advantages in fixed dose combinations of tramadol and diclofenac, in particular. Thus also taking account the safety and efficacy profile of the NSAID class, where diclofenac is clinically associated with the second lowest relative risk, ⁽¹¹⁾ and its potency substantially greater than several other agents, a fixed dose combination of tramadol, paracetamol and diclofenac, is proposed in this invention.

A vast number of receptors, biochemical transmitters and physiological processes are involved in the response and sensation of pain. Many pharmacological modalities target one specific site in order to attempt to reduce the pain symptom, and therefore do not provide satisfactorily adequate pain relief.

Nociceptive pain is pain that has a known or obvious source, such as trauma or arthritis. Neuropathic pain is defined by the International Association for the Study of Pain as pain that is initiated or caused by a primary lesion or dysfunction in the nervous system, and may be central or peripheral. Pain signals due to noxious stimuli such as inflammatory insults are converted into electrical impulses in the tissue nociceptors that are found within dorsal root ganglions. Nociceptive and neuropathic pain signals utilize the same pain pathways. The intensity, quality and location of the pain are conveyed to the sensory cortex from the somatosensory thalamus.

During persistent pain the inter-neurons in the dorsal horn release endogenous opioids in order to reduce the perceived pain. Exogenously administered opioids are thought to mimic the enkephalin and dynorphin effects of the µ-opioid receptors in the brain and spinal cord. They act peripherally on injured tissue to reduce inflammation, on the dorsal horn to impede nociceptive signal transmission and at the supraspinal level, where they activate inhibitory pathways of spinal nociceptive processing. Opioids are powerful analgesic drugs that are used as an adjunctive treatment in addition to paracetamol or NSAIDs.

Tramadol [30% water solubility; pKa 9.41; elimination half-life (t_{1/2}) 6 hours] is a weak µ- and κ-opioid receptor agonist and acts on the monoamine receptors of the autonomous nervous system preventing nor-adrenaline reuptake and displacing stored 5-HT. The synergy of its opioid and monoaminergic activity results in its analgesic activity in moderate to severe pain. It is clinically associated with fewer adverse events and a lower addictive potential, thought to be due to its binary mechanism of action, than the traditional opioids and is effective for various types of post-operative pain. In order to reduce the occurrence of adverse effects associated with opioid analgesics, they are often combined with non-opioid agents to reduce the amount of opioid needed to result in equivalent analgesia. Thus, tramadol is commonly prescribed in low-dose formulations in combination with paracetamol or non-steroidal anti-inflammatory drugs (NSAIDs). The addition of a NSAID to tramadol may also result in synergistic anti-nociception.

Paracetamol [1.4% water solubility at 20°C; pKa 9.5; elimination half-life (t_{1/2}) 1 to 3 hours], a para-aminophenol derivative, has central anti-nociceptive effects involving serotonin and serotinergic descending inhibitory pathways. It is used for its analgesic and anti-pyretic properties in mild to moderate pain and fever, and as an adjunct to opioids in the management of severe pain. It is an agent known for its excellent antipyretic effectiveness and safety profile. Dependence and tolerance are not considered a limitation in the use of non-opioid analgesics, but there is a ceiling of efficacy, above which an increase in dose provides no further therapeutic effect. In rheumatic conditions the weak anti-inflammatory activity of paracetamol limits its contribution to pain management, usually requiring the anti-inflammatory effects of the NSAIDs. The addition of an NSAID to paracetamol has been shown to improve post-operative pain treatment.

Non-steroidal anti-inflammatory drugs, (such as diclofenac, a phenylacetic acid derivative), are anti-pyretic and analgesics with central and peripheral effects. They act by inhibiting cyclo-oxygenase (COX) enzymes and synthesizing prostaglandin E₂ in traumatized and inflamed tissue, thereby increasing the threshold of activation of nociceptors. They exert anti-inflammatory effects due to their acidic character and extensive protein binding. The capillary leakage of plasma proteins and the acidic pH in the extracellular space of inflamed tissue, allows NSAIDs to concentrate in the injured tissue and exert their effects. As surgical trauma initiates peripheral inflammatory reactions that result in pain, NSAIDs are an effective post-operative option. Diclofenac [0,187% water solubility at pH 6.8; pKa 4.0; terminal plasma half-life (t_{1/2}) 1 to 2 hours] is an analgesic, antipyretic and anti-inflammatory agent that is extensively used in the long-term symptomatic treatment of rheumatoid arthritis and osteoarthritis and for the short-term treatment of acute musculoskeletal injuries, post-operative pain and dysmenorrhoea.

The administration of NSAIDs with opioids has been shown to reduce post-operative opioid consumption, allow an earlier return of post-operative bowel function and reduce the incidence of bladder spasm. In the management of severe visceral pain, analgesia seems less amenable to NSAID therapy but combination with opioids may achieve good results. The fixed combination of tramadol and paracetamol in Tramacet^{™} (Janssen-Cilag Ltd.) has proved to be a therapeutic advantage and the efficacy of both these active pharmaceutical ingredients seems to benefit from the addition of a NSAID according to the above-cited research. United States Patent 5,516,803 describes the super-additive advantage gained by combining tramadol and a NSAID and two other patents describe advantages in fixed dose combinations of tramadol and diclofenac, in particular. Thus also taking account the safety and efficacy profile of the NSAID class, where diclofenac is clinically associated with the second lowest relative risk, and its potency substantially greater than several other agents, an oral rate-modulated, site-specific pharmaceutical dosage form comprising a fixed dose combination of tramadol, paracetamol and diclofenac, is proposed.

The acronym "API" when used in this specification is intended to refer to an active pharmaceutical ingredient and to its synonym, a pharmaceutically active ingredient.

WO 2008/015221 relates to an orally disintegrating tablet comprising at least two discrete layers, one of which comprises at least one active agent that promotes the oxidation of opioids and the other of which contains granules including an inert core which is coated with at least one opioid and at least one binder, wherein said opioid coating is coated with a subcoat comprising a compound soluble in gastric fluids, said subcoat being coated with a taste-masking coating.

WO 2004/054571 relates to an oral pharmaceutical preparation, suitable for dosing every 24 hours, comprising a substrate, which substrate comprises a pharmaceutically effective amount of tramadol or a salt thereof and a pharmaceutically effective amount of topiramate and wherein said substrate may be coated with a controlled release coating.

WO 08/097066 relates to an oral sustained-release triple layer tablet consisting of an inner immediate-release layer containing a pharmaceutically active ingredient and two outer layers containing swellable polymers.

WO 2005/030181 relates to a sustained release dosage from comprising an immediate release component and a sustained release component.

### OBJECT OF THE INVENTION

It is an object of this invention to provide a multi-configured pharmaceutical dosage form and, more particularly, to provide a multi-layered tablet pharmaceutical dosage form or various multi-unit formulations suitable for the rate-modulated delivery of single or multiple pharmaceutical compositions.

### SUMMARY OF THE INVENTION

In accordance with this invention there is provided an orally ingestible three layered tablet for the delivery of at least one active pharmaceutical ingredient (API) or the pharmaceutically active salts and isomers thereof, to the gastrointestinal tract, wherein each layer includes an API, or the pharmaceutically active salts and isomers thereof, and the API are tramadol, paracetamol and diclophenac and wherein each API is integrated into a platform formed from at least one polymer and, where appropriate, excipients, which in use inhibit release of an API in a region of the gastrointestinal tract other than the desired absorption location and, thus, facilitate release of the API in a rate controlled manner when in the desired absorption location and the polymer or polymers provide, in use, first-order release kinetics of each API or the pharmaceutically active salts and isomers thereof from one or both outer layers of the tablet and wherein the tablet has a middle layer of rate-modulating polymeric material and at least one cross linking agent to provide, in use, zero-order release of the API or the pharmaceutically active salts and isomers thereof.

There is further provided for the or each API to be integrated, preferably by mixing or blending, into a platform formed from at least one and preferably a matrix of polymers and, where appropriate, excipients which, in use, inhibit release of an API in a region of the gastrointestinal tract other than the desired absorption location and facilitate release of the API in a rate controlled manner when in the desired absorption location.

There is further provided for the or each API, or the pharmaceutically active salts and isomers thereof, to be mixed with one or more excipients having a known chemical interaction such as crosslinking, dissolution rate of pH dependency, erodibility and/or swellability so that, in use, the or each API, or the pharmaceutically active salts and isomers thereof, can be released over a desired period of time, preferably in a rate-controlled manner which may be rapid alternatively slowly.

There is further provided for the polymer or polymers used in the pharmaceutical dosage form to be one or more of: a standard hydrophilic polymer or polymers, a hydrophilic swellable and/or erodible polymer or polymers, a standard hydrophobic polymer or polymers, a hydrophobic swellable and/or erodible polymer or polymers, and, preferably, one or more polymers selected from the group consisting of:: hydroxyethylcellulose (HEC), hydroxypropylcellulose (HPC), hydroxypropylmethylcellulose (HPMC), polyethylene oxide (PEO), polyvinyl alcohol (PVA), sodium alginate, pectin, ethylcellulose (EC), poly(lactic) co-glycolic acids (PLGA), polylactic acids (PLA), polymethacrylates, polycaprolactones, polyesters and polyamides, and for the polymer or polymers to be mixed with a co-polymer or used alone in the pharmaceutical dosage form.

There is also provided for the polymer or polymers to impart, to the API, or the pharmaceutically active salts and isomers thereof, in use, a phasic drug release profile and thus a time-controlled release of the or each API, preferably tramadol and paracetamol, or the pharmaceutically active salts and isomers thereof, which is released first and which is absorbed in the operatively upper regions of the gastrointestinal tract and zero-order release kinetics for an API, preferably diclofenac, or the pharmaceutically active salts and isomers thereof, which is released second and which is absorbed in a lower portion of the gastrointestinal tract.

There is further provided for the polymer or polymers to provide, in use, first-order release kinetics of one or more APIs, preferably tramadol and paracetamol, or the pharmaceutically active salts and isomers thereof, fro m a first outer layer or a tabletised dosage form having three layers and zero-order release kinetics of an API, preferably tramadol and paracetamol, or the pharmaceutically active salts and isomers thereof, from a second outer layer of the tabletised dosage form.

There is further provided for the polymer or polymers to provide, in use, first-order release kinetics of the or each APIs, preferably tramadol and paracetamol, or the pharmaceutically active salts and isomers thereof, from one or both outer layers of the tabletised dosage form which has three layers.

There is also provided for the pharmaceutically active composition/s to be from among an analgesic combination, preferably paracetamol, tramadol and diclofenac, and for each or a combination of at least two of the pharmaceutically active composition/s to be incorporated into at least one tablet-like layer that is mixed with various polymeric permutations and pharmaceutical excipients that are able to control the release of the said pharmaceutically active composition/s, or alternatively have the same alternating polymeric permutations and pharmaceutical excipients in each layer. The said pharmaceutically active composition/s may, for example, in the case of paracetamol and tramadol, or may not demonstrate synergistic therapeutic activity.

The invention extends to a method of manufacturing a pharmaceutical dosage form as described above comprising mixing a polymer in various concentrations, a pharmaceutical excipient, preferably a desired crosslinking agent and a lubricant, such as, for example, magnesium stearate, and at least one API or the pharmaceutically active salts and isomers thereof, to form at least one of layer of a number, preferably three, of layers in the pharmaceutical dosage form, for the or each layer to be dimensioned and configured so that, in use an API is released therefrom over a desired period of time and preferably in a rate-controlled manner which may be rapid alternatively slowly as a result of variations in the polymeric materials employed, pharmaceutical excipients, chemical interactions such as crosslinking that may be *in situ,* and/or diffusion path-lengths created.

There is also provided for the pharmaceutical dosage form to have at least one outer layer and, in addition to this, a middle or inner layer of rate-modulating polymeric material, preferably selected from the group consisting of polyethylene oxide and alginates, and at least one crosslinking reagent, preferably ,zinc gluconate, to provide, in use, zero-order release kinetics of an API, preferably diclofenac, or the pharmaceutically active salts and isomers thereof.

There is also provided for the outer layers of the dosage form to include a rate-modulating polymeric material, preferably polymeric material from among the group consisting of hydroxyethylcellulose, sodium starch glycollate, pregelatinised starch, powdered cellulose, maize starch and magnesium stearate, to provide, in use, first-order release kinetics of one or more APIs, preferably tramadol and paracetamol, or the pharmaceutically active salts and isomers thereof.

There is further provided for the dosage form to be tabletised and for the or each polymer to be selected to provide, in use, selected delivery profiles of the or each API from each tabletised layer, preferably in a zero-order manner from a central layer, and phasic release from two outer tablet-like layers if the said pharmaceutical dosage form comprises a total of three layers thus providing, in use, therapeutic blood levels similar to those produced by individual multiple smaller doses.

There is also provided for the API or APIs to be a combination of analgesics, preferably paracetamol, tramadol and diclofenac, and for each or a combination of at least two of the APIs to be incorporated into at least one tablet-like layer that is mixed with various polymeric permutations and pharmaceutical excipients that are able to control the release of the said pharmaceutically active composition/s, or alternatively have the same alternating polymeric permutations and pharmaceutical excipients in each layer. The said pharmaceutically active composition/s may or may not demonstrate synergistic therapeutic activity.

### DESCRIPTION OF PREFERRED EMBODIMENTS

The above and additional features of the invention will now be described and exemplified below with reference to the following non-limiting examples in which:
- Figure 1:: is a schematic diagram illustrating the formulation approaches for a) the layered tablet configuration and b) the monolithic matrix system;
- Figure 2:: shows a typical chromatographic profile of combined API HPLC analysis;
- Figure 3:: is a graph showing typical dissolution profiles of paracetamol obtained with various cellulose polymers at pH 6.8;
- Figure 4:: is a graph showing typical dissolution profiles of tramadol hydrochloride obtained with various cellulose polymers at pH 6.8;
- Figure 5:: is a graph showing typical dissolution profiles of diclofenac potassium obtained with various cellulose polymers at pH 6.8;
- Figure 6:: is a photograph of a combined API, cellulose polymer dosage form undergoing dissolution at pH 6.8;
- Figure 7:: is a photograph showing the swollen polymeric outer layers of the dosage form when submersed in water
- Figure 8:: Typical dissolution profiles of the three APIs obtained with a monolithic matrix tablet at pH 6.8
- Figure 9:: is a graph showing typical dissolution profiles of the three APIs obtained with a triple layered tablet with diclofenac potassium in the inner layer at pH 6.8
- Figure 10:: is a graph showing typical dissolution profiles of the three APIs obtained with a triple layered tablet with diclofenac potassium in the outer layer at pH 6.8.
- Figure 11:: is a graph showing typical dissolution profiles of paracetamol obtained with various crosslinked polymers at pH 6.8
- Figure 12:: is a graph showing typical dissolution profiles of tramadol hydrochloride obtained with various crosslinked polymers at pH 6.8
- Figure 13:: is a graph showing typical dissolution profiles of diclofenac potassium obtained with various crosslinked polymers at pH 6.8
- Figure 14:: Typical dissolution profiles of the three APIs reflecting polymers HEC (90.6mg) and HPC (181.25mg) reduced by 50% at pH 6.8
- Figure 15:: is a graph showing typical dissolution profiles of the three APIs reflecting alginate (12.5mg) and zinc gluconate (6.25mg) in the PEO (50mg) layer 3 at pH 6.8
- Figure 16:: is a graph showing typical dissolution profiles of the three APIs reflecting polymers HEC (45.31mg) and HPC (90.6mg) reduced by 50 % in layers 1 and 2 at pH 6.8
- Figure 17:: is a graph showing typical dissolution profiles of the three APIs reflecting polymers HEC (45.31mg) and HPC (90.6mg) in layers 1 and 2 respectively as well as the inclusion of sago (128.16mg) in layer 1 at pH 6.8
- Figure 18:: is a graph showing typical dissolution profiles of the three APIs reflecting polymers HEC (45.31mg) and HPC (90.6mg) in layers 1 and 2 respectively as well as the inclusion of sago (128.16mg in layer 1 and 150.8mg in layer 2) at pH 6.8
- Figure 19:: is a graph showing typical dissolution profiles of the combined APIs in simulated gastric fluid pH 1.2 without pepsin
- Figure 20:: is a graph showing typical dissolution profiles of the three APIs reflecting polymers HEC (22.6mg) and HPC (45.31mg) reduced by 50% and PEO (50mg) in layer 3 at pH 6.8
- Figure 21:: is a graph showing typical dissolution profiles of the three APIs reflecting polymers HEC (22.6mg) and HPC (45.31mg) and PEO increased to 75 mg (alginate increased to 18.75mg) at pH 6.8
- Figure 22:: is a graph showing typical dissolution profiles of the three APIs reflecting polymers HEC and HPC at 45.31 mg and 90.6mg respectively and the inclusion of sago in layers 1 and 2 (64.08 and 75.4 mg respectively) and PEO remaining at 50 mg at pH 6.8
- Figure 23:: is a graph showing typical dissolution profiles of the three APIs reflecting polymers HEC and HPC at 45.31mg and 90.6mg respectively and the inclusion of sago in layers 1 and 2 (64.08mg and 75.4mg respectively) and PEO increased to 75mg (alginate increased to 18.75mg) at pH 6.8
- Figure 24:: is a graph showing typical dissolution profiles of the three APIs reflecting polymers HEC and HPC at 27.10mg and 54.36 mg respectively and PEO at 100mg at pH 6.8
- Figure 25:: is a graph showing typical dissolution profiles of the three APIs reflecting polymers HEC and HPC increased (54.38mg and 108.72mg respectively) (granulated) and PEO increased to 200mg (blended) at pH 6.8
- Figure 26:: is a graph showing typical dissolution profiles of the three APIs reflecting polymers HEC and HPC increased (54.38mg and 108.72mg respectively) (blended) and PEO increased to 200mg (blended) at pH 6.8
- Figure 27:: is a graph showing typical dissolution profiles of the three APIs reflecting polymers HEC and HPC increased (54.38mg and 108.72mg respectively) (granulated) and PEO remained at 100mg (granulated) at pH 6.8
- Figure 28:: is a graph showing typical dissolution profiles of the three APIs reflecting polymers HEC and HPC increased (54.38mg and 108.72mg respectively) (blended) and PEO remained at 100mg (granulated) at pH 6.8
- Figure 29:: is a graph showing typical dissolution profiles of the three APIs reflecting 200mg LMW PEO at pH 6.8 over 8 hours

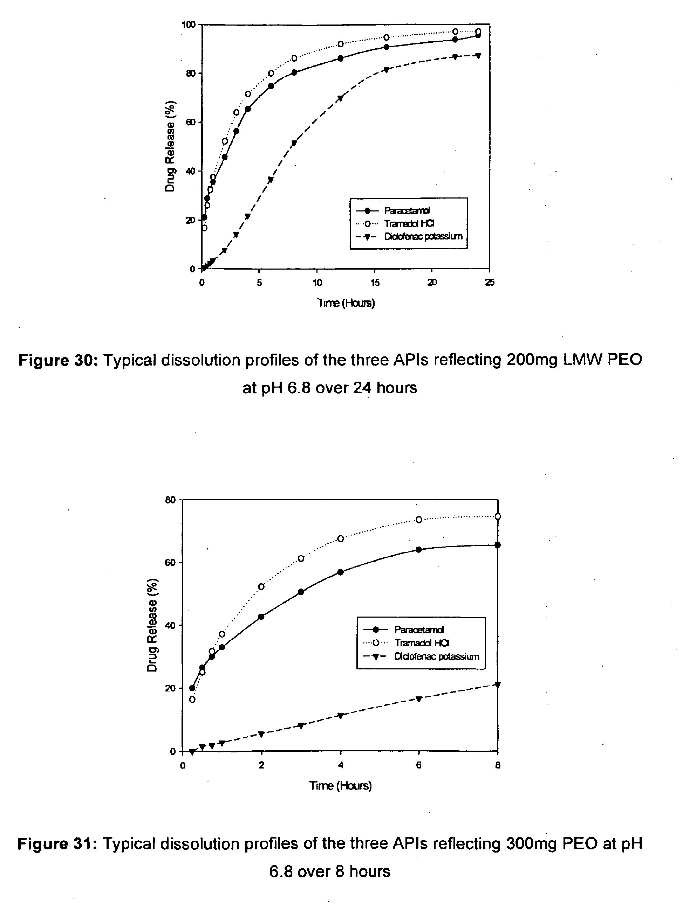

- Figure 30:: is a graph showing typical dissolution profiles of the three APIs reflecting 200mg LMW PEO at pH 6.8 over 24 hours
- Figure 31:: is a graph showing typical dissolution profiles of the three APIs reflecting 300mg PEO at pH 6.8 over 8 hours

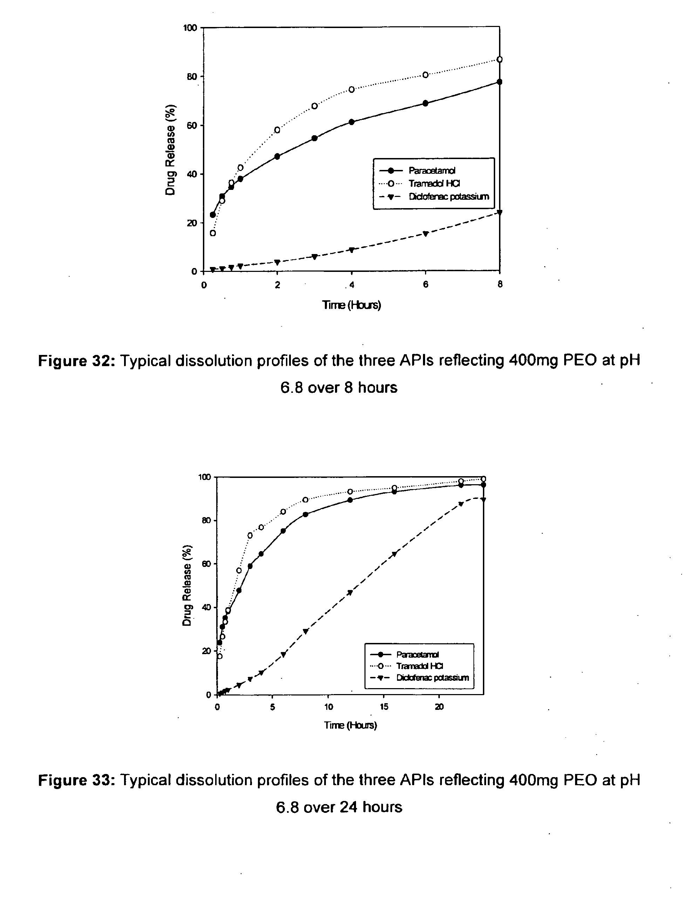

- Figure 32:: is a graph showing typical dissolution profiles of the three APIs reflecting 400mg PEO at pH 6.8 over 8 hours
- Figure 33:: is a graph showing typical dissolution profiles of the three APIs reflecting 400mg PEO at pH 6.8 over 24 hours
- Figure 34:: is a graph showing typical dissolution profiles of the three APIs reflecting 500mg PEO at pH 6.8 over 8 hours
- Figure 35:: is a graph showing typical dissolution profiles of the three APIs reflecting PEO in the outer layers at pH 6.8 over 24 hours.
- Figure 36:: is a graph showing typical dissolution profiles of the three APIs reflecting PEO and Iginate/zinc gluconate in the outer layers at pH 6.8 over 24 hours.
- Figure 37:: is a graph showing typical dissolution profiles of the three APIs reflecting PEO and Iginate/calcium chloride in the outer layers at pH 6.8 over 24 hours.
- Figure 38:: is a graph showing typical dissolution profiles of the three APIs reflecting PEO and alginate/calcium chloride (50%) in the outer layers at pH 6.8 over 24 hours.
- Figure 39:: is a graph showing typical dissolution profiles of the three APIs each in a separate layer at pH 6.8 over 24 hours.
- Figure 40:: is a graph showing typical dissolution profiles of the three APIs each in a separate layer with PEO at pH 6.8 over 24 hours.
- Figure 41:: is a graph showing typical dissolution profiles of the three APIs each in a separate layer with PEO and alginate/zinc gluconate at pH 6.8 over 24 hours; and
- Figure 42:: is a graph showing typical dissolution profiles of the three APIs each in a separate layer with PEO and alginate/calcium chloride at pH 6.8 over 24 hours,
and in the following tables in which:
- Table 1:: provides data on the dissolution study conditions;
- Table 2:: shows data of chromatographic conditions for combined API analysis; and
- Table 3:: shows the formulae studied using APIs in a 1:2 ratio with cellulose polymers.

The examples begin with the methods employed to develop an innovative pharmaceutical dosage form for facilitating the treatment of mild to moderate pain that promotes patient compliance and simplifies prescribing without increasing the side-effects of the drugs according to the invention and also endeavours to illustrate the apparent improvements on previous studies performed in an attempt to address the delivery of pharmaceutical active composition/s for the treatment and management of pain and more particularly of polymers, excipients and dosage forms according to the invention.

### Experimental Methods

### Assay method development

The suitability of a high performance liquid chromatographic (HPLC) method was confirmed by performing linearity plots for the combined APIs. Stock solutions of the active pharmaceutical ingredients were made. A 25%, 50%, 75%, 100% and 125% solution of APIs paracetamol, tramadol hydrochloride and diclofenac potassium was produced. Samples were processed by gradient elution techniques using a Waters 2695 Alliance Separations Module and Waters 2996 Photo Diode Array detector.

### Formulation and drug dissolution studies

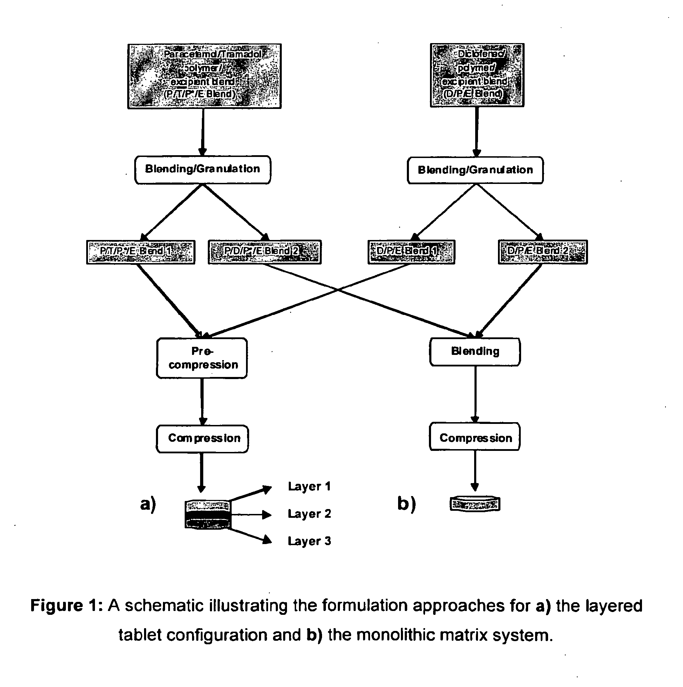

Initial dissolution characteristics of the combined APIs paracetamol, tramadol hydrochloride and diclofenac potassium; individual and combined cellulose and ethylene oxide-based polymers were determined by producing experimental batches of tablets. These were produced on a Manesty Single Punch Type F3 machine by direct compression and wet granulation techniques into monolithic matrix and multi-layered systems, as shown in Figure 1. In situ crosslinking of various alginate, pectin and eudragit polymers with salts such as zinc gluconate was also investigated for an influence on the release characteristics of the solid dosage system.

Dissolution studies were conducted using a USP rotating paddle method (Hanson Virtual Instruments SR8 Plus Dissolution Test Stations) at 50rpm in phosphate buffer pH 6.8 (900mL, 37°C±0.5°C) for each formulation employing an autosampler (Hanson Research Auto Plus Maximiser and AutoPlus^{™} MultiFill^{™}). Samples of 1.6mL were withdrawn over a period of 12 to 20 hours and analysed via HPLC. Release profiles in simulated gastric fluid pH 1.2 without pepsin over a period of four hours were determined to identify any site-specific release induced by the polymers. The dissolution studies were performed under the conditions described in Table 1.

**Table 1: Dissolution study conditions**

| | |
|---|---|
| **Apparatus** | USP Paddle Assembly |
| **Dissolution media** | a) 900mL of phosphate buffer pH 6.8. |
| | b) 900mL of simulated gastric fluid pH 1.2 without pepsin. (Preheated and maintained at 37°C±0.5°C) |
| **Speed** | 50rpm |
| **Sampling (automated)** | Autoplus Maximiser |
| **Filter (standard solution)** | Non-sterile 33mm Millex-HV Hydrophillic Durapore^{®} (PVDF) 0.45µm syringe filter unit (Millipore) |
| **Filters (test solutions)** | Hanson Research Online sample filters 10µm P/N 27-101-083 (Autoplus Maximiser) |
| **Withdrawal times** | a) 0.25; 0.5; 0.75; 1; 2; 3; 4; 8; 12; 14; 16 and 20 hours. |
| | b) 0.25; 0.5; 0.75; 1, 2, 3 and 4 hours. |

### Results and Discussion

### Assay method

The assay method developed displayed superior resolution of the API combinations and the linearity plots produced indicated that the method was sufficiently sensitive to detect the concentrations of each API over the concentration ranges studied (R²=0.99 for paracetamol, tramadol hydrochloride and diclofenac potassium). The chromatographic conditions are mentioned in Table 2.

**Table 2: Chromatographic conditions for combined API analysis.**

| | |
|---|---|
| **Column** | Atlantis T3 4.6x75mm |
| **Mobile phases** | (A) 0.1% trifluoroacetic acid pH 2.30 with 6M ammonia (pH 2.29). |
| | (B) Acetonitrile. |
| **Wavelength** | 275nm |
| **Flow rate** | 1.0mL/min |
| **Column temperature** | 15-25°C |
| **Injection volume** | 10µL |
| **Run time** | 14 minutes |

Initially paracetamol and tramadol hydrochloride showed good resolution from one another but it seemed that diclofenac potassium was retained for a longer period on the column, due to its base properties, when a run time of ten minutes was used. To overcome this, the gradient run time was increased to 14 minutes and the concentration of the organic modifier increased.

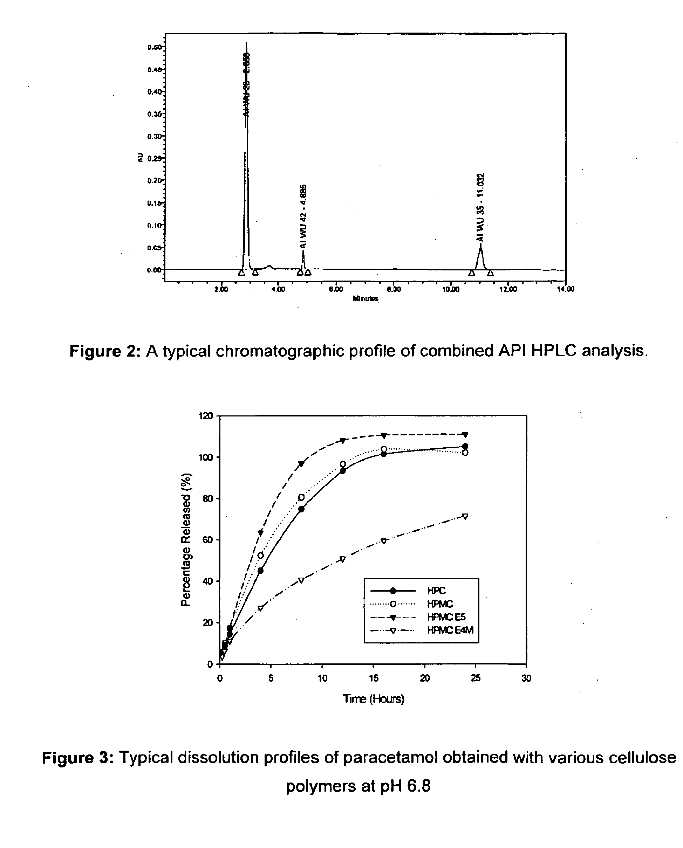

As evident in Figure 2, it is apparent that the developed method showed good resolution between each peak.

The calibration curves or linearity plots produced indicate that the method is sufficiently sensitive to detect concentrations of each of the three APIs over the concentration ranges studied. All three APIs gave linear response over the tested range. The coefficient of determination, R² or the proportion of variability in the data set is as mentioned previously. As each value is close to one, it provides assurance that the degree of goodness of fit of the linear model is satisfactory.

### Formulation and Drug Dissolution Studies

A series of experiments were performed in order to assess the pharmaceutical dosage form and attain the desired drug release profiles. These experiments are discussed hereunder.

### Experimental series one and two

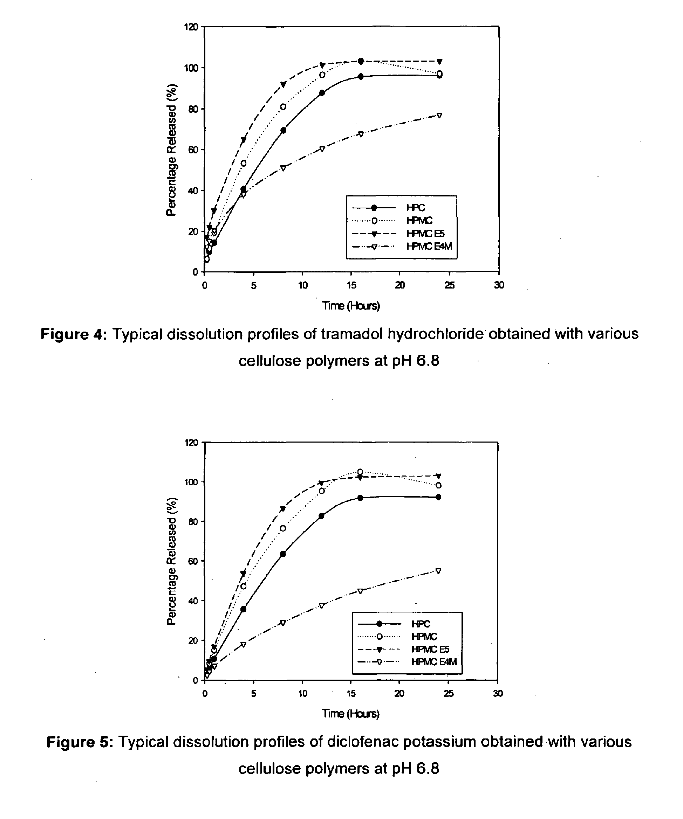

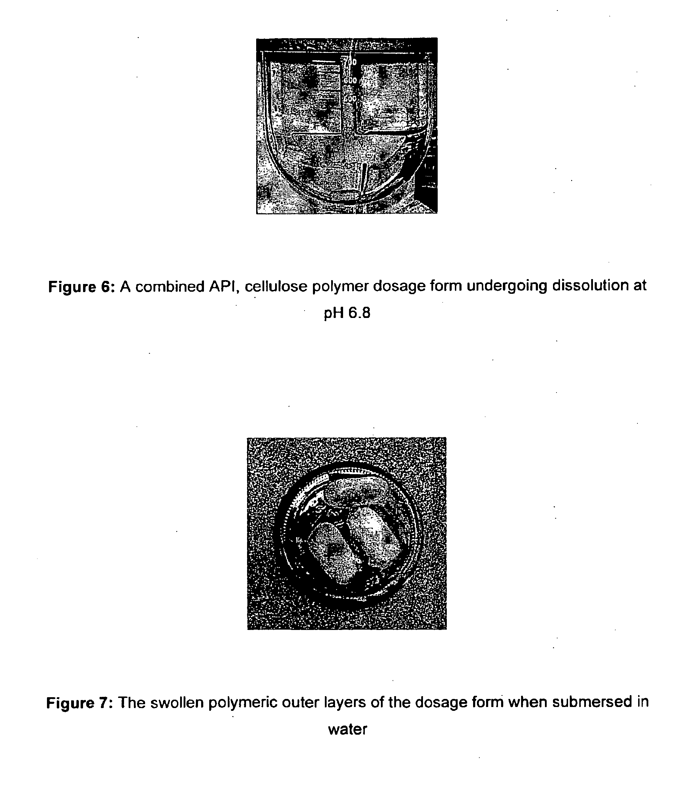

Initial dissolution characteristics of the combination of the three APIs and individual polymers were determined by producing small batches of tablets each with a different polymer. The tablets were produced using direct compression on a Manesty Single Punch Type F3 compression machine (England) fitted with 22x9mm caplet-shaped punches. The ratio of polymer to actives was kept at 2:1 with 0.5% magnesium stearate added to ensure sufficient lubrication during compression. The ingredients were blended by hand in a polyethylene bag for three minutes prior to compression. The formulae are presented in Table 3 below. The dissolution profiles obtained for each API are displayed in figures 3 to 5 below. Figures 6 and 7 demonstrate the cellulose-based polymer formulation undergoing dissolution and the release-controlling swollen outer polymeric layers of the tablet after submersion in water.

**Table 3: Formulae studied using APIs in a 1:2 ratio with cellulose polymers**

| **Quantity (mg) per tablet** | **E1/27/21A** | **E1/27/21B** | **E1/27/21C** | **E1/27/22A** | **E1/27/22B** |
|---|---|---|---|---|---|
| **Tramadol hydrochloride** | 37.5 | 37.5 | 37.5 | 37.5 | 37.5 |
| **Paracetamol** | 325 | 325 | 325 | 325 | 325 |
| **Diclofenac potassium** | 25 | 25 | 25 | 25 | 25 |
| **Polymer** | 769.18 HPC | 769.18 HEC | 769.18 HPMC (E5-LV Premium) | 769.18 HPMC (E5) | 769.18 HPMC (E4M) |
| **Magnesium stearate** | 5.813 | 5.813 | 5.813 | 5.813 | 5.813 |
| **Tablet mass** | 1162.5 | 1162.5 | 1162.5 | 1162.5 | 1162.5 |

### Experimental series three

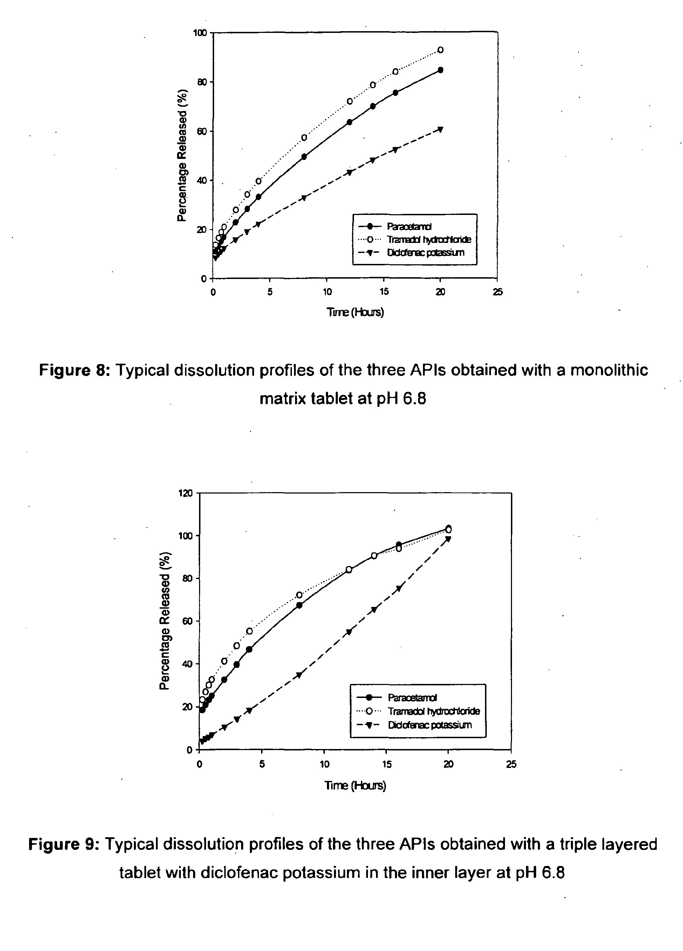

A cellulose and polyethylene oxide-based formulation was subjected to monolithic and layered tableting technology, with the three APIs demonstrating markedly different behaviour dependent solely upon their location within the dosage unit. Diclofenac potassium demonstrated both first-order and zero-order kinetics, when compressed as a monolithic matrix or layered dosage form respectively. Figures 8-10 illustrate the combined effect on the three APIs when compressed as monolithic or layered tablets.

### Experimental series four

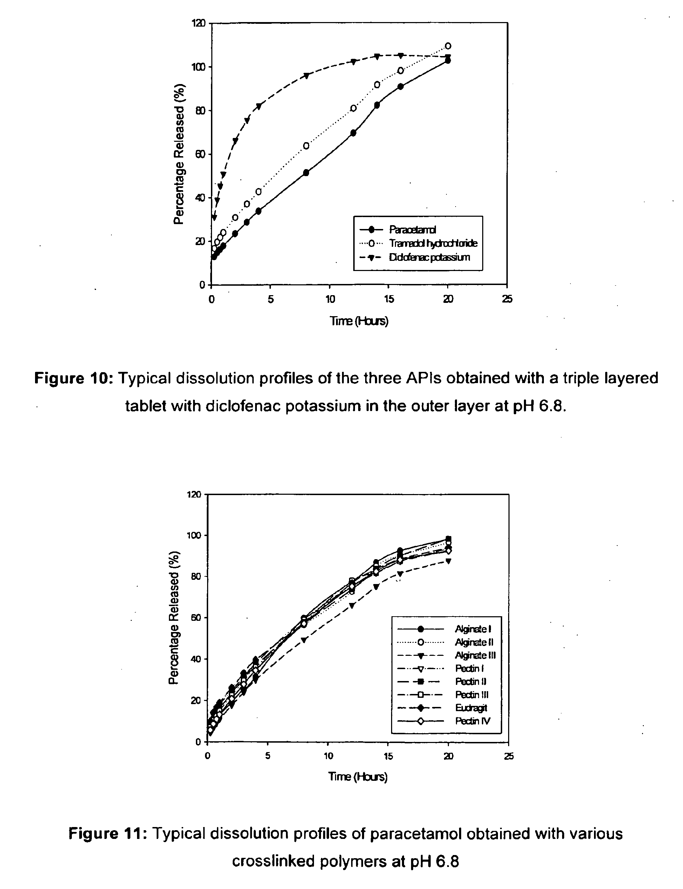

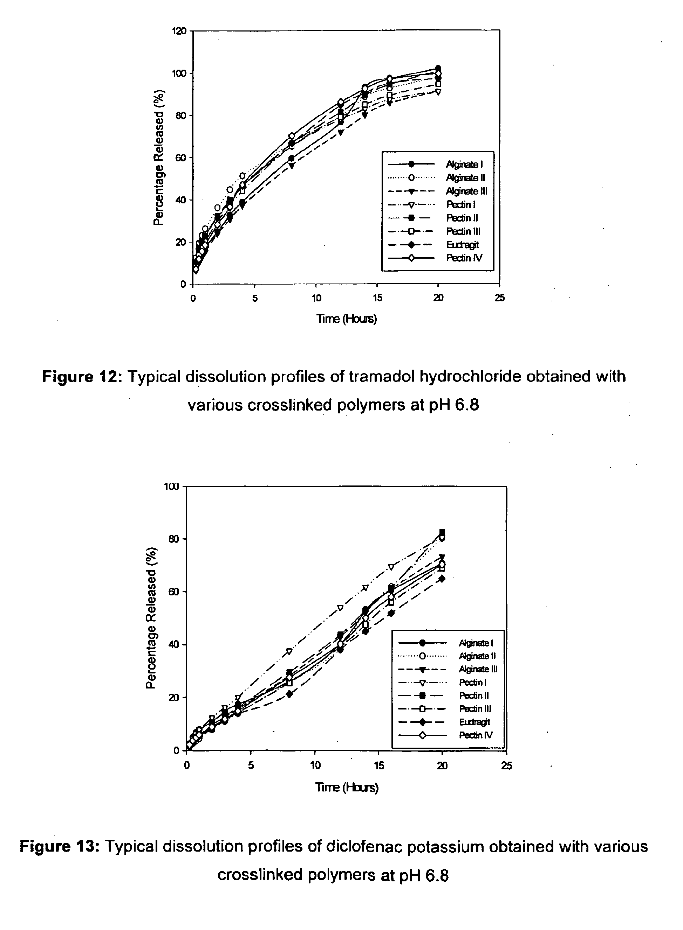

Various pectin, alginate and eudragit polymers that displayed desired *in vitro* crosslinking activity with metallic salts, were incorporated into the dosage form, to determine the effects of these polymers on the release characteristics of the combined APIs. Paracetamol and tramadol hydrochloride still showed first-order release while potassium diclofenac retained its zero-order release curve as evidenced in the release profiles in Figures 11-13 below.

### Experimental series five

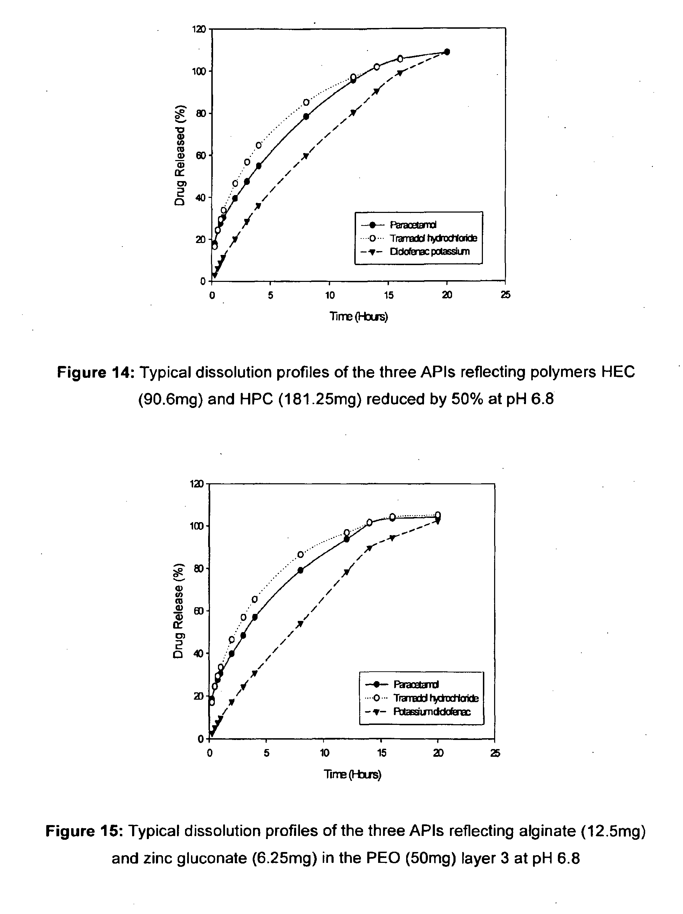

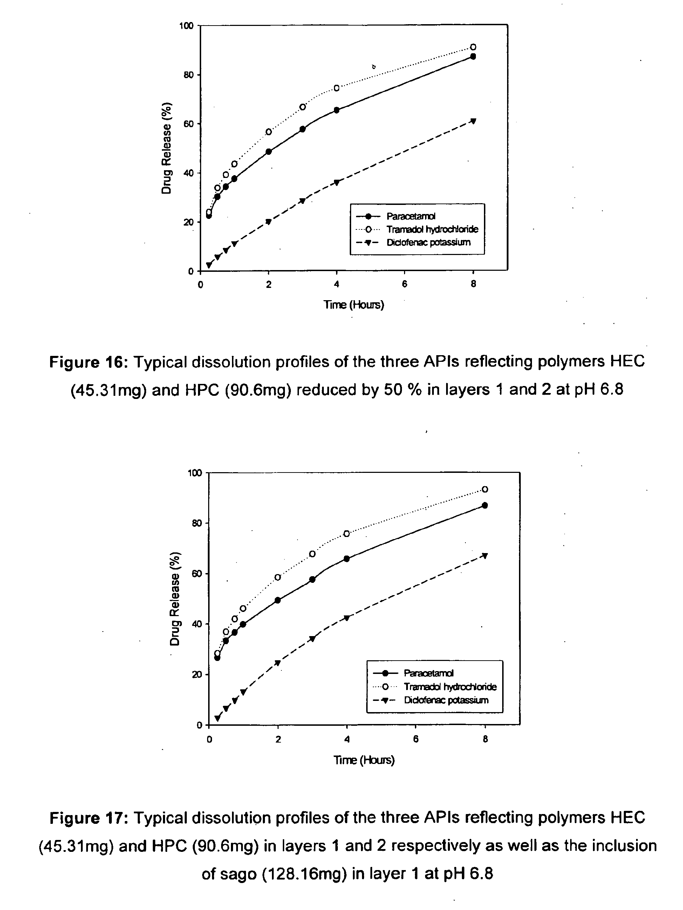

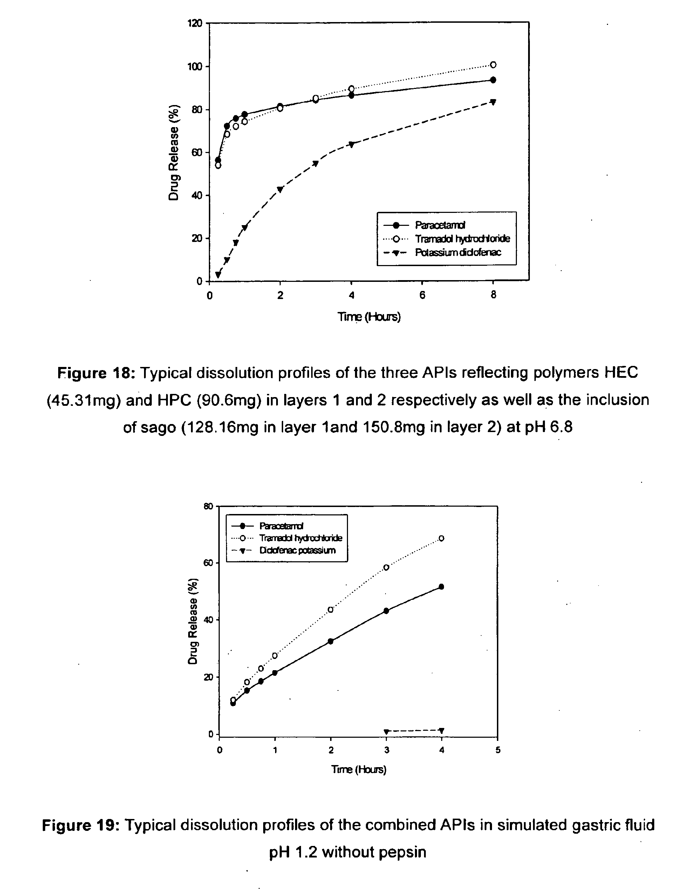

The concentration of HEC and HPC in paracetamol/tramadol layers 1 and 2 were halved to 90.6mg and 181.25mg respectively in the first formulation in this series (Figure 14). The crosslinking polymer alginate (12.5mg) and the metallic salt, zinc gluconate (6.25mg) were incorporated into the diclofenac potassium and PEO layer in the second set of experiments (Figure 15). The alginate and zinc gluconate addition was then included in a formulation where the HEC and HPC had been further reduced to 45.31 mg and 90.6mg respectively (Figure 16). To this formulation 128.16mg sago was included in paracetamol/tramadol layer 1 (Figure 17), and then both 128.16mg sago in layer 1 and 150.8mg sago in paracetamol/tramadol layer 2 (Figure 18). Figure 19 represents the formulation shown in Figure 14 run in the dissolution medium of simulated gastric fluid pH 1.2 without pepsin, to demonstrate potential site-specific release of diclofenac potassium.

### Experimental series six

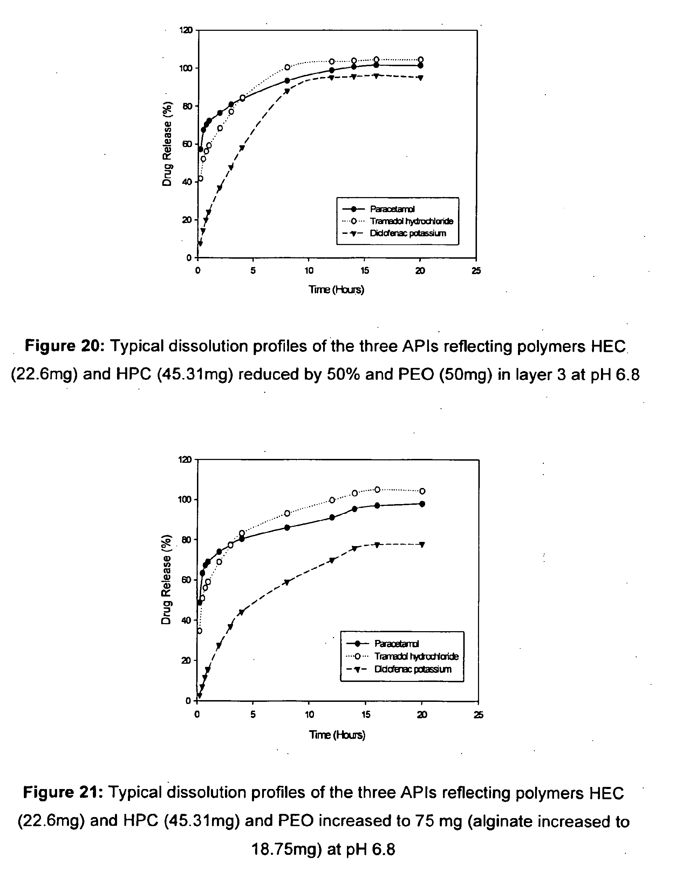

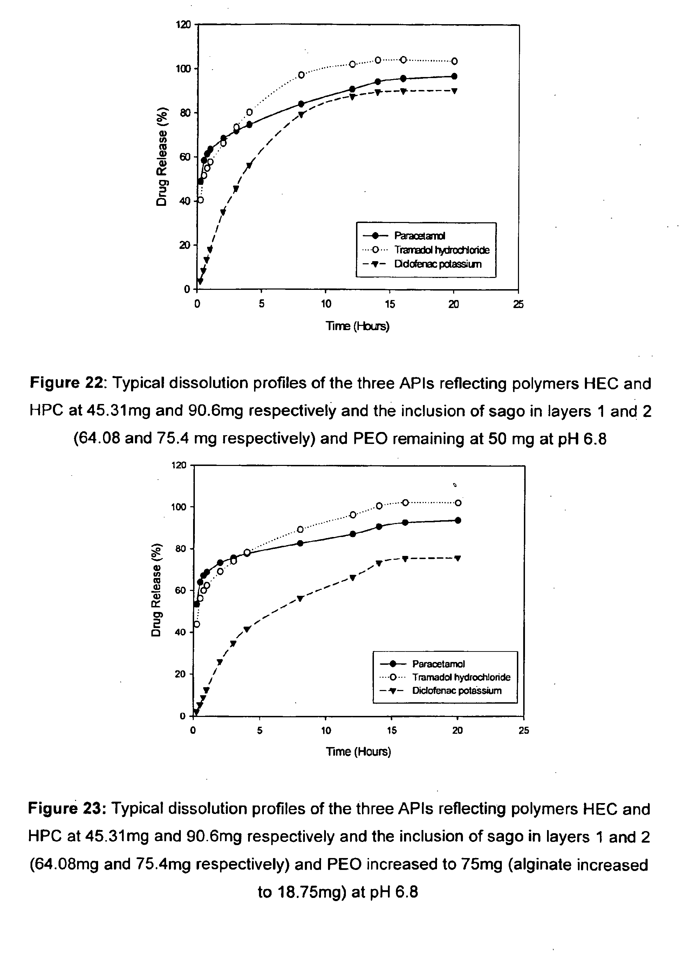

The first experiment in this series involved reducing HEC in layer 1 to 22.6mg and HPC in layer 2 to 45.31mg (Figure 20). These quantities were then included in another formulation where the PEO in layer 3 was increased to 75mg and the alginate to 18.75mg (Figure 21). The third formulation included HEC (45.31mg) and sago (64.08mg) in layer 1, HPC (90.6mg) and sago (75.4) in layer 2 and the PEO inlayer 3 was kept at 50mg (Figure 22). The final experiment in this series used the layer 1 and 2 described in formulation 3 and for layer 3 PEO was increased to 75mg, with alginate at 18.75mg and zinc gluconate at 6.25mg (Figure 23). The effect on the dissolution profiles is evident in the figures below.

### Experimental series seven

This formulation reduced the HEC in layer 1 to 27.10mg and the HPC in layer 2 to 54.36mg while the PEO in layer 3 was increased to 100mg. The alginate in layer 3 remained at 12.5mg.

### Experimental series eight

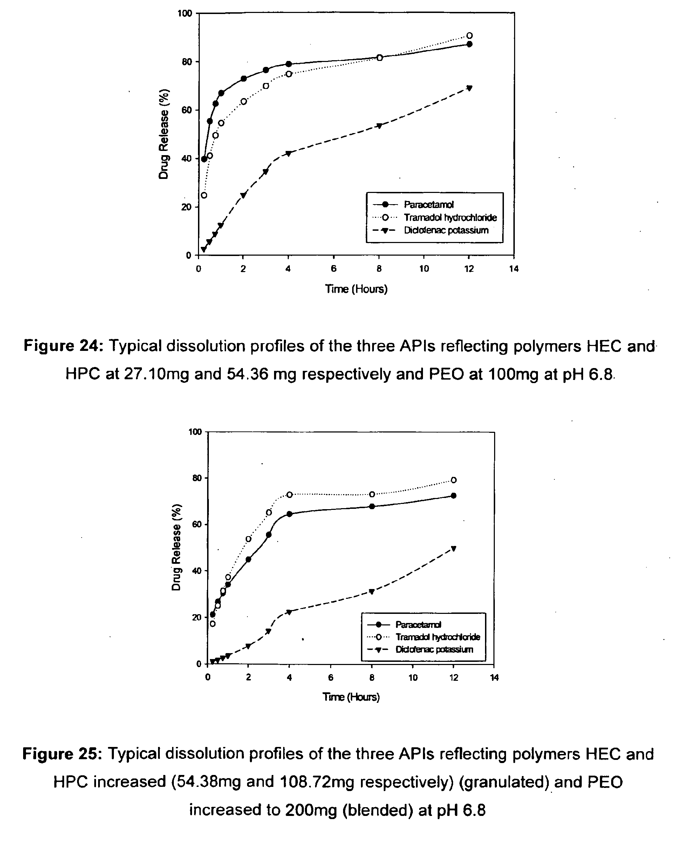

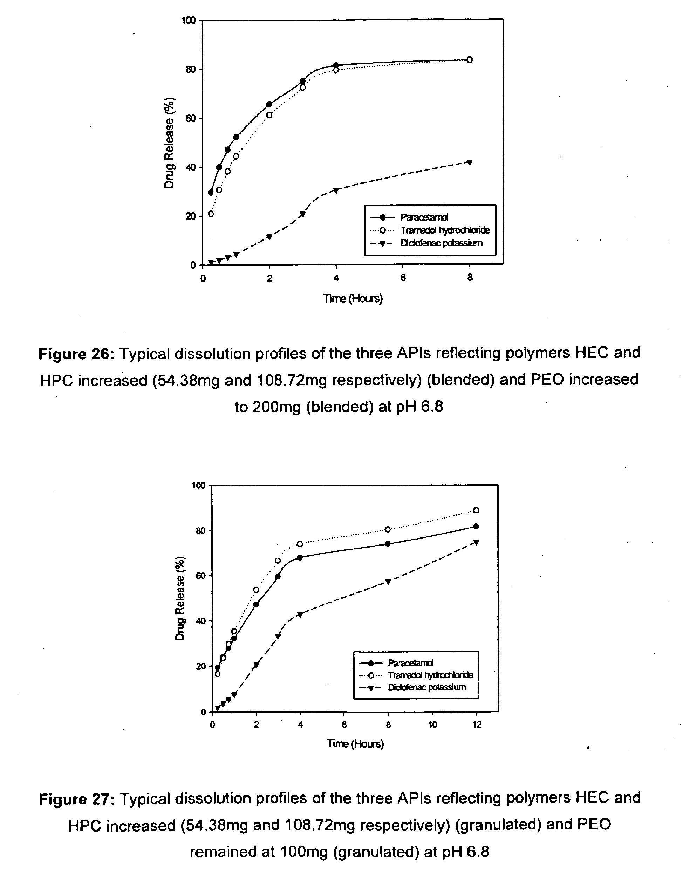

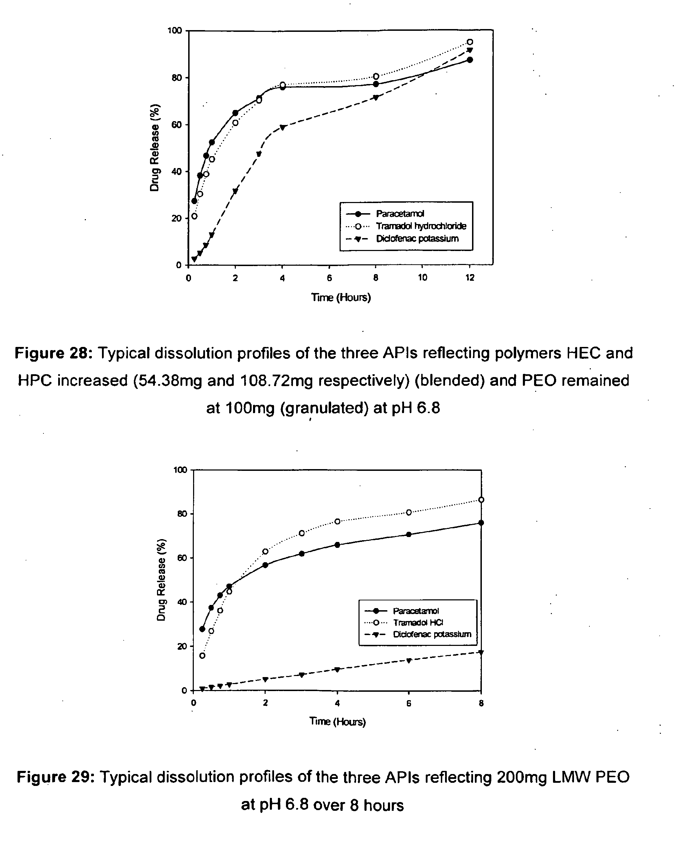

The polymer concentration in layer 1 and 2 was increased by a factor of two (HEC=54.38mg and HPC=108.72mg) to slow the release rate slightly and make it more site specific and the PEO was increased to 200mg/tablet to improve zero-order release. Dissolutions were performed over a period of 12 hours. The first experiment increased PEO to 200mg per tablet, with layer 3 being blended and layers 1 and 2 granulated (Figure 25). The second formulation was as the first but all layers were blended (Figure 26). In the third and fourth experiments, the quantities in layers 1 and 2 remained as above but the PEO in layer 3 was kept at 100mg per tablet. The diclofenac potassium, alginate and zinc gluconate for these two experiments were granulated with alcohol prior to the PEO being included. The third experiment displayed the effect of all three of the mentioned layers being granulated (Figure 27) and the fourth experiment demonstrated the effect of granulating the third layer and blending layers 1 and 2 (Figure 28).

### Experimental series nine

The quantity of polyethylene oxide in the diclofenac potassium layer was increased to 300mg, 400mg, and 500mg to see the effect on the zero-order diclofenac profile. The 200mg polyethylene oxide experiment was repeated with the lower molecular weight material (WSR301). The 200mg and 400mg experiment were run over both 8 hours and 24 hours to visualise the release effect over a 24 hour period.

The incorporation of assorted cellulose-based polymers on the typical release response of combinations of paracetamol, diclofenac potassium and tramadol hydrochloride resulted in each API displaying slight differences in their release response to the cellulose polymers implying possible rate modulating activity. The release profiles of each API obtained with various cellulose-based polymers were similar despite differing solubilities, indicating that the polymers were influential in controlling drug release.

A cellulose and polyethylene oxide-based formulation was subjected to monolithic and layered tableting technology, with the three APIs demonstrating markedly different behaviour dependent solely upon their location within the dosage unit. Diclofenac potassium demonstrated both first-order and zero-order kinetics, when compressed as a monolithic matrix or layered dosage form respectively.

Various pectin, alginate and eudragit polymers that displayed desired *in vitro* crosslinking activity with metallic salts were incorporated into the dosage form, to determine the effects of these polymers on the release characteristics of the combined APIs. Paracetamol and tramadol hydrochloride showed first-order release while diclofenac potassium retained its zero-order release curve.

In order to establish the potential site-specific release potential of the polymeric dosage form, formulations consisting of cellulose, polyethylene oxide and alginate polymers were subjected to dissolution studies in simulated gastric fluid pH 1.2 without pepsin. Typical results from these studies, shown in Figure 18, confirmed that diclofenac potassium was not released in this medium, thus its desired, site-specific release, had been obtained.

### Experimental series ten

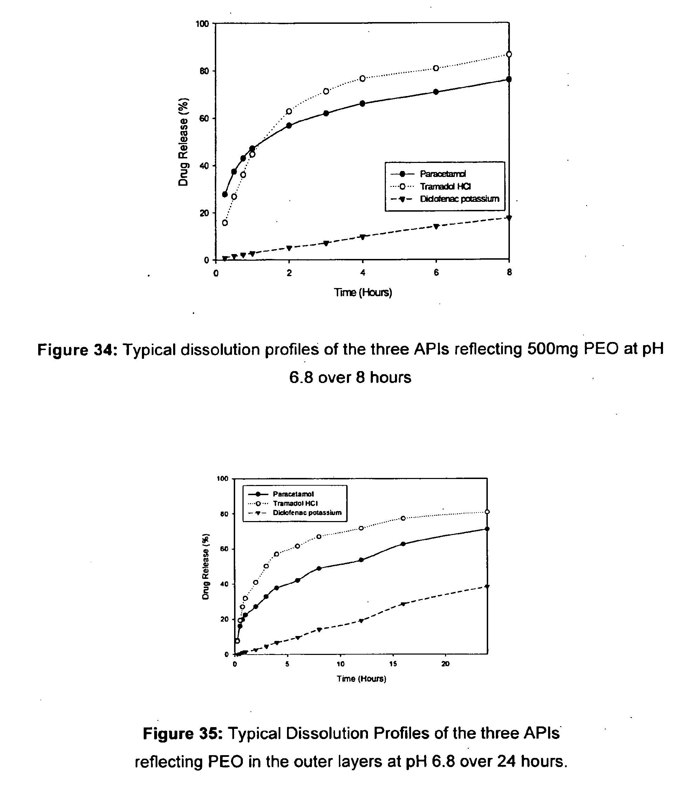

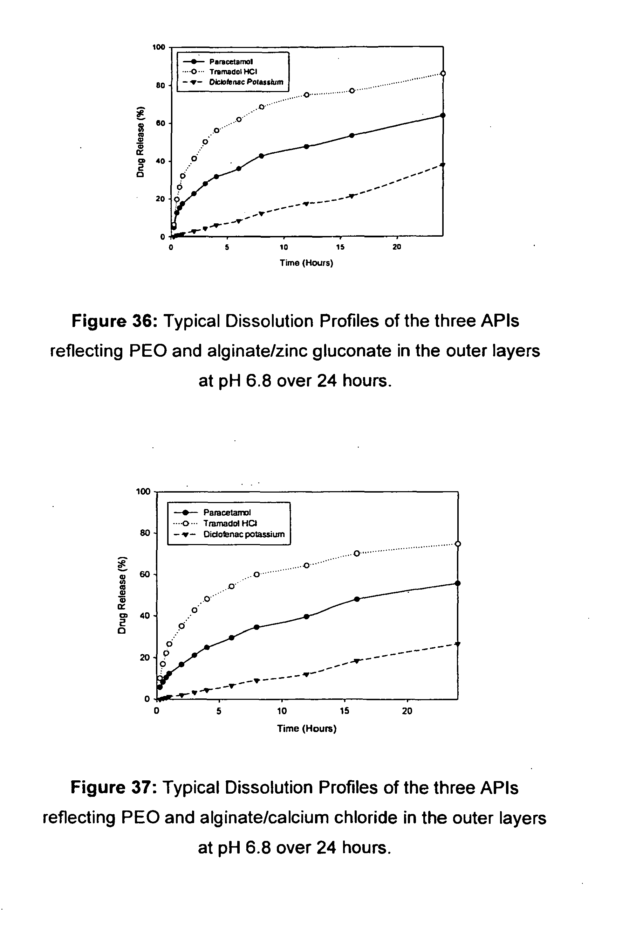

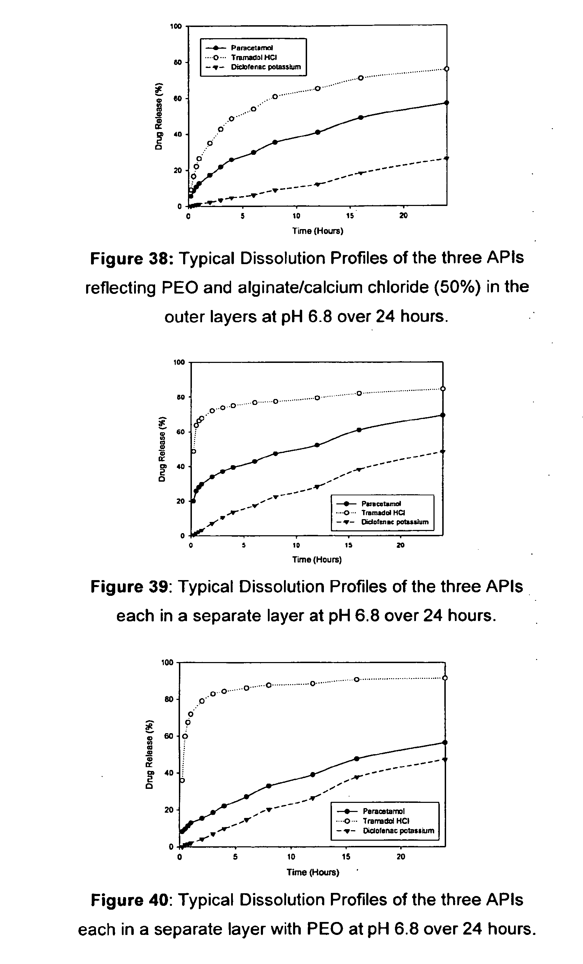

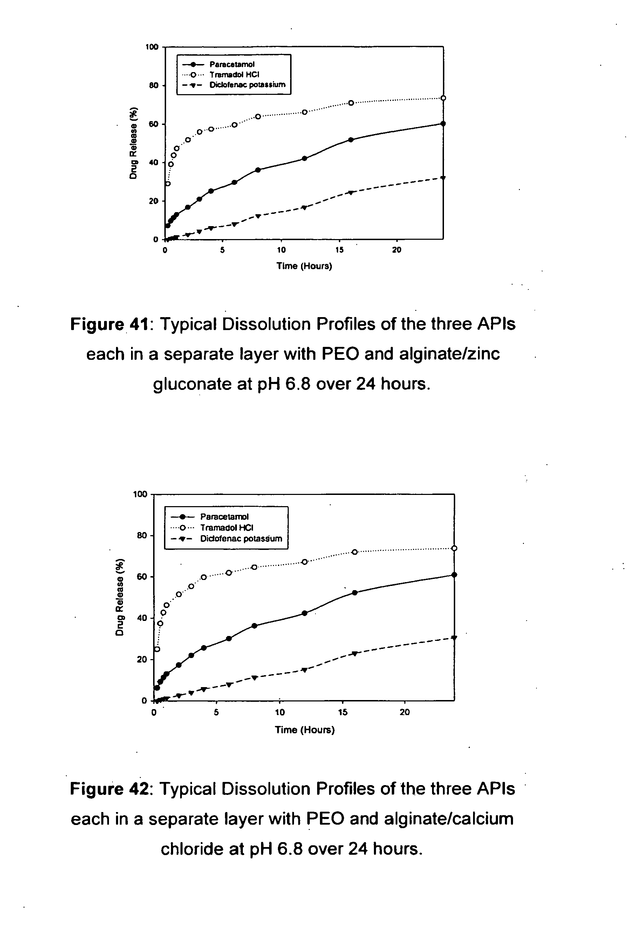

An additional number of experimental formulations were run based on the previous formulation containing 400 mg PEO. In formulation A the HEC in layer 1 was reduced to 5.12% and PEO included at 15.37% in order to keep the proportion of polymer in layer 1 constant. Layer 2, the other outer layer, was adjusted to include 8.5% HPC and 25.5% PEO. The diclofenac layer remained unchanged in this experimental series. Formulation B displayed the dissolution profile when alginate and zinc gluconate, as well as the PEO, were included in layers 1 and 2 and formulation C calcium chloride instead of zinc gluconate was used as the metallic cross-linker. Formulation D was the same as that for C but with the calcium chloride concentration halved. It was also necessary to determine the effect of having 100 % of the paracetamol in the one outer layer and 100 % of the tramadol HCl in the second outer layer. Formulation E explored this with the original concentrations of HEC and HPC used in combination with paracetamol and tramadol HCl respectively and formulation F was used to display the effect of including PEO in these outer layers. Formulation G and H were performed to display the effect of the addition of alginate and zinc gluconate and alginate and calcium chloride respectively to these layers. The dissolution profiles are displayed below in Figures 35 to 42.

### Conclusions

The assay method developed displayed superior resolution of the API combinations and the linearity plots produced indicated that the method was sufficiently sensitive to detect the concentrations of each API over the concentration ranges studied (R²=0.99 for paracetamol and R²=0.99 for tramadol hydrochloride). The dissolution profiles obtained with cellulose and ethylene oxide-based polymers displayed flexible yet rate-modulating drug release kinetics for each API. Typical first-order release kinetics was obtained from the monolithic configurations over a period of 20 hours. In addition, the application of multi-layered tableting technology allowed for the attainment of both prolonged first-order (n≥0.5) and desirable zero-order (n>0.9) release kinetics.

In addition to the above description, this invention also provides for the delivery of a wide range of other drugs within various drug classes that may or may not be administered as a combination or as a fixed dose combination, which includes but not limited to, anti-inflammatory agents, analgesic agents, anti-histamines, local anesthetics, bactericides and disinfectants, vasoconstrictors, haemostatics, chemotherapeutics, antibiotics, cosmetics, antifungals, vasodilators, antihypertensives, anti-emetics, antimigraine, anti-arrhythmics, anti-asthmatics, antidepressants, peptides, vaccines, hormones, anti-proton pumps, H-receptor blockers or lipid-lowering agents. Examples of potential drug combinations may include but are not limited to, [Antiretrovirals], [neomycin and bacitracin]; [amoxicillin and clavulanic acid]; [imipenem and cilastatin]; [sulfamethoxazole and trimethoprim]; [isoniazid and ethambutol]; [rifampicin and isoniazid]; [rifampicin, isoniazid and pyrazinamide]; [thiacetazone and isoniazid]; [benzoic acid and salicylic acid]; [ethinylestradiol and levonorgestrel]; [ethinylestradiol and levonorgestrel]; [ethinylestradiol and norethisterone]; [levodopa and carbidopa]; [ferrous salt and folic acid]; [sulfadoxine and pyrimethamine]; [lidocaine and epinephrine]; [oral rehydration salts: sodium chloride, trisodium citrate dehydrate, potassium chloride, and glucose]; [lipid-lowering agents and antihypertensives]; [sodium alendronate, colecalciferol, and calcium gluconate]; [furosemide, potassium chloride, and carvedilol]; [colchicine, diclofenac, and prednisolone].

### References

1. Galluzzi KE. Management of neuropathic pain. JAOA, 105 4 (9), 2005.
2. Camu F. Pharmacology of systemic analgesics. Best Prac. Res. Clin. Anaesth. 16 (4), 2002.
3. Rubin BR. Management of osteoarthritic knee pain. JAOA, 105 4(9), 2005.
4. Alfonso M, Goldenheim P, Sackler R. Formulation for respiratory tract administration. US Patent 6,642,275, 2003.
5. Sweetman SC. Martindale: The complete drug reference, 34th Ed, 32-33, 76-78, 94-95, 2005.
6. Jung Y, Kim DK, Kim M, Kim H, Cha I, Lee E. Onset of analgesia and analgesic efficacy of tramadol/acetaminophen and codeine/acetaminophen/ibuprofen in acute postoperative pain: A single-center, single-dose, randomized, active-controlled, parallel-group study in a dental surgery pain model. Clin. Ther. 26 (7), 2004.
7. Raffa RB. Pharmacology of oral combination analgesics: Rational therapy for pain. J. Clin. Pharm. Ther. 26, 2001.
8. Reza S, Quadir MA, Haider SS. Comparative evaluation of plastic, hydrophobic and hydrophilic polymers as matrices for controlled-release drug delivery. J Pharm. Pharmaceut. Sci., 6 (2), 2003.
9. Hardman JG. Chapter 27, Analgesic-antipyretic and anti-inflammatory agents. Goodman and Gilman's, The Pharmacological Basis of Therapeutics, 9th Ed, 637, 1996.
10. Torres LM. Paracetamol-tramadol combination. Exp. and Clin. Pharmacol. 26 (Suppl. A), 2004.
11. Breivik EK, Barkvoll P, Skovlund E. Combining diclofenac with acetaminophen or acetaminophen-codeine after oral surgery: A randomised, double-blind single-dose study. Clin. Pharmacol. Ther. 66 (6), 1999.
12. Wilder-Smith CH, Hill L, Dyer RA, Torr G, Coetzee E. Postoperative sensitization and pain after caesarean delivery and the effects of single IM doses of tramadol and diclofenac alone and in combination. Anaesthesia and Analgesia, 97, 2003.
13. Raffa RB. Composition comprising a tramadol material and a non-steroidal anti-inflammatory drug. US Patent 5,516,803, 1996.
14. Bartholomaeus J, Ziegler I. Multilayer tablet for administering a fixed combination of tramadol and diclofenac. US Patent 6,558,701, 2003.
15. Bartholomaeus, J, Kugelmann H. Parenteral dosage forms comprising a suspension of tramadol salt and diclofenac salt. US Patent 6,875,447, 2005.
16. Aulton ME. Chapter 13, Preformulation. Pharmaceutics. The Science of Dosage Form Design, International Student Edition, 249-251, 1996.

## Claims

1. An orally ingestible three layered tablet for the delivery of at least one active pharmaceutical ingredient (API) or the pharmaceutically active salts and isomers thereof, to the gastrointestinal tract, wherein each layer includes an API, or the pharmaceutically active salts and isomers thereof and the APIs are tramadol, paracetamol and diclophenac and wherein each API is integrated into a platform formed from at least one polymer and, where appropriate, excipients, which, in use, inhibit release of an API in a region of the gastrointestinal tract other than the desired absorption location and, thus, facilitate release of the API in a rate controlled manner when in the desired absorption location and the polymer or polymers provide, in use, first-order release kinetics of each API or the pharmaceutically active salts and isomers thereof from one or both outer layers of the tablet and wherein the tablet has a middle layer of rate-modulating polymeric material and at least one cross linking agent to provide, in use, zero-order release of the API or the pharmaceutically active salts and isomers thereof.

2. A tablet according to claim 1 wherein the or each polymer is in the form of a matrix.

3. A tablet according to claim 1 or 2 wherein the APIs are tramadol and paracetamol.

4. A tablet as claimed in any one of claims 1 to 3 in which the or each API, or the pharmaceutically active salts and isomers thereof, is or are mixed with one or more excipients having a known chemical interaction such as crosslinking, dissolution rate of pH dependency, erodibility and/or swellability so that, in use, the or each API, or the pharmaceutically active salts and isomers thereof, can be released over a desired period of time, preferably in a rate-controlled manner which may be rapid alternatively slowly.

5. A tablet as claimed in claim 1 in which the polymer or polymers used in the tablet is or are one or more of: a standard hydrophilic polymer or polymers, a hydrophilic swellable and/or erodible polymer or polymers, a standard hydrophobic polymer or polymers, a hydrophobic swellable and/or erodible polymer or polymers.

6. A tablet as claimed in claim 5 in which the polymer or polymers is or are selected from the group consisting of: hydroxyethylcellulose (HEC), hydroxypropylcellulose (HPC), hydroxypropylmethylcellulose (HPMC), polyethylene oxide (PEO), polyvinyl alcohol (PVA), sodium alginate, pectin, ethylcellulose (EC), poly(lactic) co-glycolic acids (PLGA), polylactic acids (PLA), polymethacrylates, polycaprolactones, polyesters and polyamides.

7. A tablet as claimed in claim 5 or in claim 6 in which, the polymer or polymers is or are mixed with a co-polymer or used alone in the tablet.

8. A tablet as claimed in any one of the preceding claims in which the polymer or polymers impart, to the API, or the pharmaceutically active salts and isomers thereof, in use, a phasic drug release profile and thus a time-controlled release of the or each API, or the pharmaceutically active salts and isomers thereof, which is released first and which is absorbed in the operatively upper regions of the gastrointestinal tract and zero-order release kinetics for an API, or the pharmaceutically active salts and isomers thereof, which is released second and which is absorbed in a lower portion of the gastrointestinal tract.

9. A tablet as claimed in any one of the preceding claims in which the or each API is incorporated into at least one tablet-like layer and is mixed with various polymeric permutations and pharmaceutical excipients that are able to control the release of the or each API.

10. A tablet as claimed in claim 9 in which the tablet-like layers of the tablet have the same alternating polymeric permutations and pharmaceutical excipients in each layer.

11. A tablet as claimed in any one of the preceding claims in which the tablet incorporates two or more APIs which may or may not demonstrate synergistic therapeutic activity.

12. A tablet as claimed in claim 11 in which the APIs demonstrate a synergistic therapeutic activity.

13. A method of manufacturing a tablet as claimed in any one of the preceding claims comprising mixing a polymer in various concentrations, a pharmaceutical excipient, optionally a desired crosslinking agent and a lubricant and at least one API or the pharmaceutically active salts and isomers thereof selected from tramadol, paracetamol and diclophenac, to form at least one layer of three layers in the tablet, dimensioning and configuring the or each layer.

14. A method of manufacturing a tablet as claimed in claim 13 in which the tablet is provided with at least one outer layer and, in addition to this, a middle or inner layer of rate-modulating polymeric material, and at least one crosslinking reagent, to provide, in use, zero-order release kinetics of an API or the pharmaceutically active salts and isomers thereof.

15. A method of manufacturing a tablet as claimed in claim 14 in which the outer layers of the dosage form include a rate-modulating polymeric material, preferably polymeric material from among the group consisting of hydroxyethylcellulose, sodium starch glycollate, pregelatinised starch, powdered cellulose, maize starch and magnesium stearate, to provide, in use, first-order release kinetics of one or more APIs, or the pharmaceutically active salts and isomers thereof.

16. A method of manufacturing a tablet as in claim 15 which includes selecting the or each polymer to be selected to provide, in use, selected delivery profiles of the or each API from each tabletised layer, preferably in a zero-order manner from a central layer, and phasic release from two outer tablet-like layers thus providing, in use, therapeutic blood levels similar to those produced by individual multiple smaller doses.

17. A method of manufacturing a tablet as claimed in any one of claims 13 to 16 in which each API or combination of at least two of the APIs is incorporated into at least one tablet-like layer that is mixed with various polymeric permutations and have the same alternating polymeric permutations and pharmaceutical excipients are present in each layer.

18. A method of manufacturing a tablet as claimed in any one of claims 13 to 17 in which the API or APIs may or may not demonstrate synergistic therapeutic activity.

## Patentansprüche

1. Oral einnehmbare, drei-schichtige Tablette zum Abgeben wenigstens eines aktiven, pharmazeutischen Bestandteils (API) oder der pharmazeutisch aktiven Salze und Isomere hiervon an den gastrointestinalen Trakt, wobei jede Schicht einen API, oder die pharmazeutisch aktiven Salze und Isomere hiervon, enthalt und die APIs Tramadol, Paracetamol und Diclofenac sind, und wobei jeder API in eine Plattform integriert ist, die gebildet ist aus wenigstens einem Polymer und, wenn angebracht, Hilfsstoffen, die im Gebrauch die Abgabe eines API in einer von dem gewünschten Absorptionsort verschiedenen Region des gastrointestinalen Trakts hemmen und somit die Abgabe des API in einer ratenkontrollierten Weise am gewünschten Absorptionsort erleichtern, und wobei das Polymer oder die Polymere im Gebrauch eine Abgabekinetik erster Ordnung jedes API oder der pharmazeutisch aktiven Salze und Isomere hiervon aus einer oder beiden äußeren Schichten der Tablette bereit stellen, und wobei die Tablette eine mittlere Schicht aus einem ratenmodulierenden polymeren Material und wenigstens einem quervernetzenden Agens enthält, um im Gebrauch eine Abgabe nullter Ordnung des API oder der pharmazeutisch aktiven Salze und Isomere hiervon bereit zu stellen.

2. Tablette nach Anspruch 1, bei welcher das oder jedes Polymer in Form einer Matrix vorliegt.

3. Tablette nach Anspruch 1 oder 2, bei welcher die APIs Tramadol und Paracetamol sind.

4. Tablette nach einem der Ansprüche 1 bis 3, bei welcher der oder jeder API, oder die pharmazeutisch aktiven Salze und Isomere hiervon, mit einem oder mehreren Hilfsstoffen gemischt ist oder sind, welche eine bekannte chemische Wechselwirkung wie Quervernetzung, Auflösungsrate in pH-Abhängigkeit, Abtragbarkeit und/oder Quellfähigkeit haben, so dass im Gebrauch der oder jeder API, oder die pharmazeutisch aktiven Salze und Isomere hiervon, über einen gewünschten Zeitraum abgegeben werden können, vorzugsweise in einer ratenkontrollierten Weise, die schnell oder alternativ langsam sein kann.

5. Tablette nach Anspruch 1, bei welcher das in der Tablette verwendete Polymer oder die Polymere eines oder mehrere ist oder sind aus: ein standardmäßiges hydrophiles Polymer oder Polymere, ein hydrophiles quellfähiges und/oder abtragbares Polymer oder Polymere, ein standardmäßiges hydrophobes Polymer oder Polymere, ein hydrophobes quellfähiges und/oder abtragbares Polymer oder Polymere.

6. Tablette nach Anspruch 5, bei welcher das Polymer oder die Polymere aus der Gruppe gewählt ist oder sind, welche besteht aus: Hydroxyethylzellulose (HEC), Hydroxypropylzellulose (HPC), Hydroxypropylmethylzellulose (HPMC), Polyethylenoxid (PEO), Polyvinylalkohol (PVA), Natriumalginat, Pektin, Ethylzellulose (EC), Poly(Milch-) coglykolsäuren (PLGA), Polymilchsäuren (PLA), Polymethacrylate, Polycaprolaktone, Polyester und Polyamide.

7. Tablette nach Anspruch 5 oder Anspruch 6, bei welcher das Polymer oder die Polymere mit einem Co-Polymer gemischt ist oder sind, oder alleine in der Tablette verwendet sind.

8. Tablette nach einem der vorherigen Ansprüche, bei welcher das Polymer oder die Polymere dem API, oder den pharmazeutisch aktiven Salzen und Isomeren hiervon, im Gebrauch ein phasisches Wirkstoffabgabeprofil und somit eine zeitkontrollierte Abgabe des oder jedes API, oder der pharmazeutisch aktiven Salze und Isomere hiervon, der zuerst abgegeben und in den funktionell oberen Regionen des gastrointestinalen Trakts absorbiert wird, und eine Abgabekinetik nullter Ordnung für einen API, oder der pharmazeutisch aktiven Salze und Isomere hiervon, der als zweiter abgegeben und in einem tieferen Bereich des gastrointestinalen Trakts absorbiert wird, verleihen.

9. Tablette nach einem der vorherigen Ansprüche, bei welcher der oder jeder API in wenigstens eine tablettenartige Schicht eingebracht wird und mit verschiedenen polymeren Permutationen und pharmazeutischen Hilfsstoffen, die in der Lage sind, die Abgabe des oder jedes API zu kontrollieren, gemischt ist.

10. Tablette nach Anspruch 9, bei welcher die tablettenartigen Schichten der Tablette dieselben alternierenden polymeren Permutationen und pharmazeutischen Hilfsstoffe in jeder Schicht aufweisen.

11. Tablette nach einem der vorherigen Ansprüche, bei welcher die Tablette zwei oder mehr APIs, die eine synergistische therapeutische Aktivität zeigen können oder nicht, enthält.

12. Tablette nach Anspruch 11, bei welcher die APIs eine synergistische therapeutische Aktivität zeigen.

13. Verfahren zum Herstellen einer Tablette nach einem der vorherigen Ansprüche, umfassend Mischen eines Polymers in verschiedenen Konzentrationen, eines pharmazeutischen Hilfsstoffs, optional eines gewünschten quervernetzenden Agens und eines Gleitmittels und wenigstens eines API oder pharmazeutisch aktiven Salze und Isomere hiervon, gewählt aus Tramadol, Paracetamol und Diclophenac, zum Formen wenigstens einer Schicht von drei Schichten in der Tablette, Dimensionieren und Konfigurieren der oder jeder Schicht.

14. Verfahren zum Herstellen einer Tablette nach Anspruch 13, bei welchem die Tablette mit wenigstens einer äußeren Schicht und, zusätzlich hierzu, einer mittleren und inneren Schicht aus einem ratenmodulierenden polymeren Material und wenigstens einem quervernetzenden Agens versehen wird, um im Gebrauch eine Abgabekinetik nullter Ordnung eines API oder der pharmazeutisch aktiven Salze und Isomere hiervon zur Verfügung zu stellen.

15. Verfahren zum Herstellen einer Tablette nach Anspruch 14, bei welchem die äußeren Schichten der Dosierform ein ratenmodulierendes polymeres Material, vorzugsweise ein polymeres Material aus der Gruppe, bestehend aus Hydroxyethylzellulose, Natrium-Stärke-Glykolat, vorgelierte Stärke, gepulverte Zellulose, Maisstärke und Magnesiumstearat, enthalten, um im Gebrauch eine Abgabekinetik erster Ordnung von einem oder mehreren APIs, oder der pharmazeutisch aktiven Salze und Isomere hiervon, bereit zu stellen.

16. Verfahren zum Herstellen einer Tablette nach Anspruch 15, umfassend das Auswählen des oder jedes Polymers, so gewählt, um im Gebrauch ausgewählte Abgabeprofile des oder jedes API aus jeder tablettisierten Schicht, vorzugsweise in einer Weise der nullten Ordnung aus einer zentralen Schicht, zu erhalten, und im Gebrauch eine phasige Abgabe aus zwei äußeren tablettenartigen Schichten zu erhalten mit therapeutischen Blutpegeln ähnlich zu jenen, die durch einzelne mehrfache kleinere Dosen erzeugt werden.

17. Verfahren zum Herstellen einer Tablette nach einem der Ansprüche 13 bis 16, bei welchem jeder API oder eine Kombination aus wenigsten zwei der APIs in wenigstens eine tablettenartige Schicht eingebracht wird, die mit verschiedenen polymeren Permutationen gemischt wird und dieselben alternierenden polymeren Permutationen haben und wobei pharmazeutische Hilfsstoffe in jeder Schicht vorliegen.

18. Verfahren zum Herstellen einer Tablette nach einem der Ansprüche 13 bis 17, bei welchem die API oder APIs eine synergistische therapeutische Aktivität zeigen oder nicht.

## Revendications

1. Comprimé à trois couches ingérable par voie orale pour l'administration d'au moins un ingrédient pharmaceutique actif (API) ou de ses sels pharmaceutiquement actifs et isomères, au système gastro-intestinal, dans lequel chaque couche comprend un API, ou ses sels pharmaceutiquement actifs et isomères et les API sont le tramadol, le paracétamol et le diclofénac et dans lequel chaque API est intégré dans une plate-forme formée à partir d'au moins un polymère et, lorsque c'est approprié, d'excipients, qui, en utilisation, inhibent la libération d'un API dans une région du système gastro-intestinal autre que l'emplacement souhaité de l'absorption et, ainsi, facilitent la libération de l'API dans un mode de vitesse contrôlée lorsqu'il se trouve dans l'emplacement souhaité de l'absorption et le polymère ou les polymères fournissent, en utilisation, une cinétique de libération de premier ordre de chaque API ou de ses sels pharmaceutiquement actifs et isomères à partir de l'une ou des deux couches externes du comprimé et où le comprimé comporte une couche moyenne de matériau polymère modulant la vitesse et au moins un agent de réticulation pour fournir, en utilisation, une libération d'ordre zéro de l'API ou de ses sels pharmaceutiquement actifs et isomères.

2. Comprimé selon la revendication 1, dans lequel le ou chaque polymère est sous la forme d'une matrice.

3. Comprimé selon la revendication 1 ou 2, dans lequel les API sont le tramadol et le paracétamol.

4. Comprimé tel que revendiqué dans l'une quelconque des revendications 1 à 3, dans lequel le ou chaque API, ou ses sels pharmaceutiquement actifs et isomères, est ou sont mélangés avec un ou plusieurs excipients présentant une interaction chimique connue comme une réticulation, une vitesse de dissolution dépendante du pH, une aptitude à l'érosion et/ou au gonflement si bien que, en utilisation, le ou chaque API, ou ses sels pharmaceutiquement actifs et isomères, peut être libéré sur une période de temps souhaitée, de préférence dans un mode de vitesse contrôlée qui peut être alternativement rapide ou lente.

5. Comprimé tel que revendiqué dans la revendication 1, dans lequel le polymère ou les polymères utilisés dans le comprimé est ou sont un ou plusieurs de : un polymère ou des polymères hydrophiles classiques, un polymère ou des polymères hydrophiles pouvant gonfler et/ou érodables, un polymère ou des polymères hydrophobes classiques, un polymère ou des polymères hydrophobes pouvant gonfler et/ou érodables.

6. Comprimé tel que revendiqué dans la revendication 5, dans lequel le polymère ou les polymères est ou sont choisis dans le groupe constitué de : hydroxyéthylcellulose (HEC), hydroxypropylcellulose (HPC), hydroxypropylméthylcellulose (HPMC), poly(oxyde d'éthylène) (PEO), poly(alcool vinylique) (PVA), alginate de sodium, pectine, éthylcellulose (EC), poly(acides lactiques-co-glycoliques) (PLGA), poly(acides lactiques) (PLA), polyméthacrylates, polycaprolactones, polyesters et polyamides.

7. Comprimé tel que revendiqué dans la revendication 5 ou dans la revendication 6, dans lequel, le polymère ou les polymères est ou sont mélangés avec un copolymère ou utilisés seuls dans le comprimé.

8. Comprimé tel que revendiqué dans l'une quelconque des revendications précédentes, dans lequel le polymère ou les polymères confèrent, à l'API, ou à ses sels pharmaceutiquement actifs et isomères, en utilisation, un profil phasique de libération des médicaments et ainsi une libération contrôlée dans le temps du ou de chaque API, ou de ses sels pharmaceutiquement actifs et isomères, qui est libéré en premier et qui est absorbé dans les régions fonctionnellement supérieures du système gastro-intestinal et une cinétique de libération d'ordre zéro pour un API, ou ses sels pharmaceutiquement actifs et isomères, qui est libéré en second et qui est absorbé dans une partie inférieure du système gastro-intestinal.

9. Comprimé tel que revendiqué dans l'une quelconque des revendications précédentes, dans lequel le ou chaque API est incorporé dans au moins une couche de type comprimé et est mélangé avec diverses permutations polymères et excipients pharmaceutiques qui sont capables de contrôler la libération du ou de chaque API.

10. Comprimé tel que revendiqué dans la revendication 9, dans lequel les couches de type comprimé du comprimé comportent les mêmes permutations polymères et excipients pharmaceutiques en alternance dans chaque couche.

11. Comprimé tel que revendiqué dans l'une quelconque des revendications précédentes, où le comprimé incorpore deux API ou plus qui peuvent démontrer ou pas une activité thérapeutique synergique.

12. Comprimé tel que revendiqué dans la revendication 11, dans lequel les API démontrent une activité thérapeutique synergique.

13. Procédé de fabrication d'un comprimé tel que revendiqué dans l'une quelconque des revendications précédentes comprenant le mélange d'un polymère dans diverses concentrations, d'un excipient pharmaceutique, éventuellement d'un agent de réticulation souhaité et d'un lubrifiant et d'au moins un API ou de ses sels pharmaceutiquement actifs et isomères choisi parmi le tramadol, le paracétamol et le diclofénac, pour former au moins une couche des trois couches dans le comprimé, en dimensionnant et en configurant la ou chaque couche.

14. Procédé de fabrication d'un comprimé tel que revendiqué dans la revendication 13, dans lequel le comprimé est fourni avec au moins une couche externe et, en plus de celle-ci, une couche moyenne ou interne de matériau polymère modulant la vitesse, et au moins un réactif de réticulation, pour fournir, en utilisation, une cinétique de libération d'ordre zéro d'un API ou de ses sels pharmaceutiquement actifs et isomères.

15. Procédé de fabrication d'un comprimé tel que revendiqué dans la revendication 14, dans lequel les couches externes de la forme galénique comprennent un matériau polymère modulant la vitesse, de préférence un matériau polymère choisi dans le groupe constitué d'hydroxyéthylcellulose, glycolate d'amidon sodique, amidon prégélatinisé, cellulose en poudre, amidon de maïs et stéarate de magnésium, pour fournir, en utilisation, une cinétique de libération de premier ordre d'un ou de plusieurs API, ou de leurs sels pharmaceutiquement actifs et isomères.

16. Procédé de fabrication d'un comprimé comme dans la revendication 15, qui comprend la sélection du ou de chaque polymère à choisir pour fournir, en utilisation, des profils d'administration choisis du ou de chaque API à partir de chaque couche transformée en comprimé, de préférence dans un mode d'ordre zéro à partir d'une couche centrale, et une libération phasique à partir des deux couches externes de type comprimé fournissant ainsi, en utilisation, des taux thérapeutiques dans le sang similaires à ceux produits par de multiples doses individuelles plus petites.

17. Procédé de fabrication d'un comprimé tel que revendiqué dans l'une quelconque des revendications 13 à 16, dans lequel chaque API ou combinaison d'au moins deux des API est incorporé dans au moins une couche de type comprimé qui est mélangée avec diverses permutations polymères et comportent les mêmes permutations polymères et excipients pharmaceutiques en alternance présents dans chaque couche.

18. Procédé de fabrication d'un comprimé tel que revendiqué dans l'une quelconque des revendications 13 à 17, dans lequel l'API ou les API peuvent démontrer ou pas une activité thérapeutique synergique.
